(19) Europäisches Patentamt
European Patent Office
Office européen des brevets

(11) EP 0 770 671 B1

(12) **EUROPEAN PATENT SPECIFICATION**

(45) Date of publication and mention of the grant of the patent:
**22.09.1999 Bulletin 1999/38**

(51) Int Cl.[6]: **C11B 9/00**, A61K 7/46

(21) Application number: **96203019.3**

(22) Date of filing: **30.10.1996**

# (54) Cyclopentylidene-cyclopentanol in perfumery

Cyclopentylidene-cyclopentanol in Parfümerie

Cyclopentylidène-cyclopentanol en parfumerie

(84) Designated Contracting States:
**CH DE ES FR GB LI NL**

(30) Priority: **03.11.1995 EP 95307853**

(43) Date of publication of application:
**02.05.1997 Bulletin 1997/18**

(73) Proprietor: **QUEST INTERNATIONAL B.V.**
**1411 GP Naarden (NL)**

(72) Inventor: **Martin, Angela**
**Ashford, Kent TN24 0LT (GB)**

(74) Representative: **Graham, John George et al**
**ICI Group Intellectual Property**
**P.O. Box 90**
**Wilton**
**Middlesbrough, Cleveland TS90 8JE (GB)**

(56) References cited:
**EP-A- 0 016 650** **WO-A-81/00845**

- **BULLETIN DE LA SOCIETE CHIMIQUE DE FRANCE, 1963, PARIS FR, pages 611-619, XP002035703 G.LE GUILLANTON: "N<SYMBOL**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 770 671 B1

Printed by Jouve, 75001 PARIS (FR)

## Description

The present invention relates to the use of 2-cyclopentylidene-cyclopentanol as fragrance and flavour material. It also relates to perfumes and perfumed products as well as flavourings and flavoured products containing this alcohol.

Many synthetic perfume components have been developed, especially in the last decades to substitute known perfume materials of natural origin. Nevertheless there is a constant need for new synthetic perfume components which are more stable than those previously developed and/or have additional or more delicate odour notes to further complete the fragrance palette from which the perfumer can chose in composing perfumes which are suitable also for various agressive environments. Thus, indole and skatole are well known fragrance materials with a strong animalic odour, often described as faecal. They are extensively used in the art of perfumery, e.g. in various floral perfume compositions, particularly in jamin perfumes and bases. However, indole and skatole suffer from the disadvantage that they are unstable to light and under alkaline conditions, such as in soap, and thus give rise to severe discoloration problems.

2-Cyclopentyl-cyclopentanol is known as a fragrance material under the tradename TELLUROL. It has a minty camphoraceous odour with a slight indolic note. It therefore has various uses in the art of perfumery, but is entirely unsuitable as a replacement for indole or skatole. 2-Cyclopentyl-cyclopentanone is described as useful as a fragrance and flavour material in EP-A-0 016 650. It has a fresh cool minty, menthone-like odour and is especially suitable for dentifrices.

It has now been found that 2-cyclopentylidene-cyclopentanol of the formula below (comprising 2 isomers):

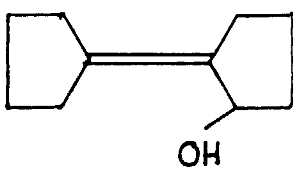

is a valuable fragrance and flavour material with a strong indolic/skatolic odour without any disturbing minty, menthone-like or camphoraceous notes. It is stable to light and in alkaline media and gives no discolouration problems. Thus, in perfumes it is an excellent substitute for indole or skatole and does not show any of the disadvantages of these known fragrance materials.

Although the alcohol according to the invention is itself known in the literature, no olfactive properties have been described before.

According to one aspect of the present invention there are provided perfumes and flavourings comprising known fragrance or flavour materials characterized in that they further contain an effective amount of 2-cyclopentylidene-cyclopentanol.

In another aspect of the invention, there are provided perfumed or flavoured products characterized in that they contain an effective amount of 2-cyclopentylidene-cyclopentanol.

In a further aspect of the invention, there is provided a process for preparing a perfume comprising the steps of reducing 2-cyclopentylidene-cyclopentanone, purifying the reaction mixture and combining the purified reaction mixture containing 2-cyclopentylidene-cyclopentanol with other fragrance materials.

The compound may be prepared as described in the literature, see: Le Guillanton et al, Bull. Soc. Chim. France **1963**(3), 611-19; Lamant et al, Compt. rend. **250**, 362-4 (1960); Mousseron et al, Bull. Soc. Chim. France **1947**, 598-605 and Mitsui et al, Bull. Chem. Soc. Japan **39**(4), 694-7 (1966). A particularly useful route starts with the self-aldol condensation of cyclopentanone, followed by reduction of the thus formed 2-cyclopentylidene-cyclopentanone. The reduction may be performed e.g. with a suitable borohydride reducing agent or according to the method of Meerwein, Ponndorf and Verley. These synthesis routes yield mixtures of the isomers and various other minor components. Apart from the usual purification steps such as distillation and/or recrystallization, these mixtures do not require further purification or separation to be used as fragrance materials in the preparation of perfumes. Thus the invention also provides a process for the preparation of a perfume comprising the steps of reducing 2-cyclopentylidene-cyclopentanone, purifying the reaction mixture and combining the purified reaction mixture with other fragrance materials.

Thus, the alcohol according to the invention may be used as such to give indole/skatole-like odour or flavour notes to all sorts of products. More particularly, it may be usefully incorporated in perfumes and flavourings. For the purposes of this invention a perfume is defined as a mixture of various fragrance materials, if desired dissolved in a suitable solvent or mixed with a solid substrate, which is used to provide a desired odour to the skin or to all sorts of products. Examples of such perfumed products are: fabric washing powders and liquids, fabric softeners and other

fabric care products; detergents, hard surface cleaners and household cleaning, scouring and disinfection products; air fresheners, room sprays and pomanders; candles; soaps, shampoos, hair conditioners, bath and shower gels and other personal care products; cosmetics such as creams, ointments, toilet waters, preshave-, aftershave- and other lotions, talcum powders, body deodorants and antiperspirants.

Known fragrance and flavour materials which may be advantageously combined with the alcohol according to the invention may be natural products such as extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carbocyclic and heterocyclic compounds. Such materials are mentioned, for example, in S. Arctander, Perfume and Flavor Chemicals (Montclair, N.J., 1969), in S. Arctander, Perfume and Flavor Materials of Natural Origin (Elizabeth, N.J., 1960), in "Flavor and Fragrance Materials - 1991", Allured Publishing Co. Wheaton, Ill. USA and in Fenaroli's Handbook of Flavor Ingredients, Vol I & II, CRC Press, Boca Raton 1995.

Examples of fragrance materials which can be used in combination with the esters according to the invention are: geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, styrallyl acetate, benzyl benzoate, amyl salicylate, dimethylbenzyl carbinol, trichloromethylphenylcarbinyl acetate, p-tert-butyl cyclohexyl acetate, isononyl acetate, vetiveryl acetate, vetiverol, α-hexylcinnamaldehyde, 2-methyl-3-(p-tert-butylphenyl)propanal, 2-methyl-3-(p-isopropylphenyl)propanal, 3-(p-tert-butylphenyl)-propanal, tricyclodecenyl acetate, tricyclodecenyl propionate, 4-(4-hydroxy-4-methyl-pentyl)-3-cyclohexenecarbaldehyde, 4-(4-methyl-3-pentenyl)-3-cyclohexenecarbaldehyde, 4-acetoxy-3-pentyltetrahydropyran, 3-carboxymethyl-2-pentylcyclopentane, 2-n-heptylcyclopentanone, 3-methyl-2-pentyl-2-cyclopentenone, n-decanal, n-dodecanal, 9-decenol-1, phenoxyethyl isobutyrate, phenyl-acetaldehyde dimethylacetal, phenyl-acetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks tetralin musks isochroman musks macrocyclic ketones, macrolactone musks ethylene brassylate, aromatic nitromusks.

Solvents which can be used in perfume compositions which contain compounds according to the invention are, for example: ethanol, isopropanol, diethylene glycol monoethyl ether, dipropylene glycol, diethyl phthalate, triethyl citrate, etc.

The amounts in which the alcohol according to the invention can be effectively used in perfumes or flavourings, or products to be perfumed or flavoured, may vary within wide limits and depend, inter alia, on the nature of the product, on the nature and the quantity of the other components of the perfume in which the compound is used and on the olfactive effect desired. It is therefore only possible to specify wide limits, which, however, provide sufficient information for the person skilled in the art to be able to use the alcohol according to the invention for his specific purpose. In perfumes an amount of 0.01% by weight or more of the alcohol according to the invention will generally have a clearly perceptible olfactive effect. Preferably the amount is 0.05% by weight, but due to the odour character of the compound it will seldom be more than 10% by weight. The amount of the alcohol according to the invention present in products will generally be at least 1 ppm by weight.

The following examples are only intended to illustrate the preparation and use of the alcohol according to the invention, but the invention is not in any way limited thereto

**Example 1**

**Preparation of 2-cyclopentylidene-cyclopentanol**

A <u>2-Cyclopentylidene-cyclopentanone.</u>

A 2 litre round bottom flask, fitted with a mechanical stirrer, a reflux condenser and a thermometer, was charged with 951g (11.3 mol) of cyclopentanone and a solution of 38.2g of potassium hydroxide in 250ml of water. The mixture was heated with stirring at about 100°C for 5 hours. After cooling to room temperature 200ml of diethyl ether was added and the aqueous and organic phases were separated. The organic phase was washed twice with 200ml of water, once with 200ml of 10% aqueous HCl solution and twice with 200ml of saturated brine solution. The organic phase was dried over anhydrous magnesium sulphate. After filtration of the organic liquid, the solvent was removed using a rotary evaporator and 813.5g of crude product was obtained. This was fractionated under reduced pressure (0.2-0.3kPa). The desired product distilled at 105-108°C and was obtained in 73% yield (623g).

B 2-cyclopentylidene-cyclopentanol

A 2 litre round bottom flask, fitted with a mechanical stirrer, a reflux condenser and a thermometer, was charged with 412.4g (2.75mol) of 2-Cyclopentylidene-cyclopentanone and 500ml methanol. The mixture was stirred until homogeneous at room temperature and then 40g of sodium borohydride was added in small portions over a period of 1.5 hour. The reaction mixture was thereafter stirred for another 4 hours. During addition as well as thereafter the temperature was kept below 40°C by cooling with a cold water bath if necessary. The reaction mixture was further stirred overnight. Thereafter it was quenched by dropwise addition, with stirring, of 250ml of water. The aqueous and organic layers were separated and the aqueous layer extracted twice with 200ml diethyl ether. The combined organic layers were washed with 200 ml water and dried over magnesium sulphate. After filtration the solvent was removed using a rotary evaporator and 444g of crude product were obtained. This was fractioned under reduced pressure (0.4kPa) through a 0.5 meter Sulzer-packed column. Fractions were collected at 90-100°C and odour assessed. The total of fractions with an acceptable odour was 182.9g. The product could be further purified by recristallization from methanol.

**Example 2**

A perfume compostion of the "White Lilac" type was prepared according to the following recipe:

| | |
|---|---|
| Phenylethylalcohol | 35.2 %w/w |
| Hydroxycitronellal | 32.0 |
| Cinnamic Alcohol | 17.5 |
| Styrax Resinoid | 7.0 |
| Anisic Aldehyde | 4.4 |
| Jasmin Absolute | 3.5 |
| 2-Cyclopentylidene-cyclopentanol | 0.4 |
| Total | 100 % |

**Example 3**

A perfume compostion of the "Orange Flower" type was prepared according to the following recipe:

| | |
|---|---|
| Phenylethylalcohol | 21.0 %w/w |
| Linalool | 21.0 |
| Petitgrain Oil | 17.0 |
| Methyl Anthranilate | 13.0 |
| Nerol 97% | 12.0 |
| Benzyl Acetate | 8.0 |
| Aurantion (Q) | 4.0 |
| Orange Flower Absolute | 2.0 |
| Aldehyde C10 (10% in diethylphthalate) | 0.8 |
| Geranyl Formate | 0.6 |
| Aldehyde C8 (10% in diethylphthalate) | 0.4 |
| 2-Cyclopentylidene-cyclopentanol | 0.2 |
| Total | 100 % |

(Q) marketed by Quest International

**Claims**

1. Perfumes and flavourings comprising known fragrance or flavour materials characterized in that they further contain an effective amount of 2-cyclopentylidene-cyclopentanol.

2. Perfumes and flavourings according to claim 1 characterized in that they contain at least 0.01% by weight of 2-cyclopentylidene-cyclopentanol.

3. Perfumed or flavoured products characterized in that they contain an effective amount of 2-cyclopentylidene-cyclopentanol.

4. Perfumed products according to claim 4 characterized in that they contain at least 1 ppm by weight of 2-cyclopentylidene-cyclopentanol.

5. Process for preparing a perfume comprising the steps of reducing 2-cyclopentylidene-cyclopentanone, purifying the reaction mixture and combining the purified reaction mixture containing 2-cyclopentylidene-cyclopentanol with other fragrance materials.

6. Process according to claim 5 characterized in that the reduction is carried out using a borohydride reducing agent.

7. Process according to claim 5 characterized in that the reaction is carried out according to Meerwein-Ponndorf-Verley.

**Revendications**

1. Parfums et arômes comprenant des matériaux connus d'odeur ou d'arôme caractérisés en ce qu'ils contiennent en plus une quantité efficace de 2-cyclopentylidène-cyclopentanol.

2. Parfums et arômes selon la revendication 1, caractérisés en ce qu'ils contiennent au moins 0,01 % en poids de 2-cyclopentylidène-cyclopentanol.

3. Produits parfumés ou aromatisés caractérisés en ce qu'ils contiennent une quantité efficace de 2-cyclopentylidène-cyclopentanol.

4. Produits parfumés selon la revendication 3, caractérisés en ce qu'ils contiennent au moins 1 ppm en poids de 2-cyclopentylidène-cyclopentanol.

5. Procédé de préparation d'un parfum comprenant les étapes de réduction de la 2-cyclopentylidène-cyclopentanone, de purification du mélange réactionnel et de combinaison du mélange réactionnel purifié contenant le 2-cyclopentylidène-cyclopentanol avec d'autres matériaux odorants.

6. Procédé selon la revendication 5, caractérisé en ce que la réduction est effectuée en utilisant un agent réducteur borohydrure.

7. Procédé selon la revendication 5, caractérisé en ce que la réaction est effectuée selon Meerwein-Ponndorf-Verley.

**Patentansprüche**

1. Parfüme und Aromastoffe, umfassend bekannte Duft- und Aromamaterialien, dadurch gekennzeichnet, daß sie ferner eine wirksame Menge an 2-Cyclopentyliden-cyclopentanol enthalten.

2. Parfüme und Aromastoffe nach Anspruch 1, dadurch gekennzeichnet, daß sie mindestens 0,01 Gewichtsprozent 2-Cyclopentyliden-cyclopentanol enthalten.

3. Parfümierte und aromatisierte Produkte, dadurch gekennzeichnet, daß sie eine wirksame Menge an 2-Cyclopentyliden-cyclopentanol enthalten.

4. Parfümierte Produkte nach Anspruch 3, dadurch gekennzeichnet, daß sie mindestens 1 Gewichts-ppm 2-Cyclopentyliden-cyclopentanol enthalten.

5. Verfahren zur Herstellung eines Parfüms, umfassend die Schritte der Reduktion von 2-Cyclopentyliden-cyclopentanon, der Reinigung der Reaktionsmischung und der Kombination der gereinigten, 2-Cyclopentyliden-cyclopentanol enthaltenden Reaktionsmischung mit anderen Duftmaterialien.

6. Verfahren nach Anspruch 5, dadurch gekennzeichnet, daß die Reduktion unter Verwendung eines Borhydridreduktionsmittels durchgeführt wird.

7. Verfahren nach Anspruch 5, dadurch gekennzeichnet, daß die Reaktion gemäß Meerwein-Ponndorf-Verley durchgeführt wird.